# EUROPÄISCHE PATENTSCHRIFT

(11) **EP 1 038 514 B1**
(45) Veröffentlichungstag und Bekanntmachung des Hinweises auf die Patenterteilung: **30.11.2005**
(21) Anmeldenummer: 00104033.6
(22) Anmeldetag: 26.02.2000
(51) Int. Cl.: A61K 7/02, A45D 40/26

(54) **Kosmetische Masse für Lippenstift, Lidstift oder Puder**
Cosmetic compositions for lipsticks, eyeliners or powders
Composition cosmétique pour rouge à lèvres, crayon à paupières ou poudre

(30) Priorität: 27.02.1999 DE 19908653
(43) Veröffentlichungstag der Anmeldung: 27.09.2000
(73) Patentinhaber: NELE Kosmetik GmbH, 91338 Igensdorf (DE)
(72) Erfinder: Hempel, Matthias, Dr., 90562 Heroldsberg (DE)
(74) Vertreter: Schneck, Herbert

(56) Entgegenhaltungen:
- FR-A- 2 330 353
- US-A- 3 957 969
- US-A- 5 180 241
- US-A- 5 192 153

## Beschreibung

Die Erfindung richtet sich auf eine kosmetische Masse für einen Lippenstift, einen Lidstift, einen Puder oder dergleichen umfassend
- Wasser mit einem Anteil von 30 bis 60%
- emulgierbares Hartwachs mit einem Anteil von 5 bis 50%
- einen mehrwertigen Alkohol als Lösungsvermittler mit einem Anteil von 15 bis 40% und vorzugsweise
einen wasserlöslichen, bis wenigstens 100°C temperaturstabilen Filmbildner mit einem Anteil von 2 bis 20%.

Derartige kosmetische Massen enthalten einerseits Pigmente und herkömmlicherweise darüber hinaus zur Erzielung einer hinreichenden Gleit- und Abstrichfähigkeit Öle, welche jedoch den Nachteil aufweisen, daß sie die Haftfähigkeit herabsetzen.

Der Erfindung liegt die Aufgabe zugrunde, eine derartige Masse zu schaffen, bei der auf die Haftfähigkeit herabsetzende Ölkomponente verzichtet werden kann.

Im Gegensatz zu herkömmlichen derartigen Massen (z.B. US 3 957 969), die lipophil eingestellt sind, ist die erfindungsgemäße Masse hydrophil ausgebildet. Aufgrund der dadurch bedingten Unverträglichkeit mit dem Hautfett wird eine extrem verbesserte Haftfähigkeit erzielt. Durch den erfindungsgemäß eingesetzten Filmbildner wird die Masse dabei wasserfest und fixiert auf der Haut in Sekunden.

Da ein solcher Filmbildner die Migration der Masse auf der Haut verhindert, werden überraschenderweise auch bei Verwendung von Diolen herkömmlicherweise zuweilen beobachtbare Hautirritationen vermieden.

Günstigerweise ist vorgesehen, daß die Hartwachse gebildet sind durch Stearamide Mea und/oder PVP/Eicosene Copolymer.

Mit Vorteil wird als Filmbildner eingesetzt Acrylates Copolymer, PVP, Hydroxyethylcellulose, vorzugsweise Diglycol/CHDM/Isophthalates/SIP Copolymer.

Der Polyalkohol kann günstigerweise ein Butylene Glycol sein.

Nachfolgend wird die Erfindung anhand zweier bevorzugter Ausführungsbeispiele beschrieben.

### 1. Ausführungsbeispiel: Eyeliner

| (Die nachfolgenden Angaben sind in INCI Nomenklatur angegeben) | |
|---|---|
| 36,50 % | Aqua |
| 17,90 % | Butylene Glycol (Polyalkohol) |
| 4,45 % | Stearamide Mea (Wachs) |
| 4,45 % | PVP (Filmbildner) |

| Als Konservierungsmittel: | |
|---|---|
| 0,50 % | Phenoxyethanol |
| 0,22 % | Methylparaben |
| 0,08 % | Buthylparaben |
| 0,05 % | Ethylparaben |
| 0,05 % | Propylparaben |

| Als Pigmente: | |
|---|---|
| 15,05 % | Mica |
| 13,95 % | Titanium Dioxide |
| 5,35 % | Iron Oxide Black |
| 1,45 % | Ferric Ferrocyanide |

### 2. Ausführungsbeispiel: Lippenstift

| (Die nachfolgenden Angaben sind in INCI Nomenklatur angegeben) | |
|---|---|
| 53,90 % | Aqua |
| 25,80 % | Butylene Glycol (Polyalkohol) |
| 6,65 % | Stearamide Mea (Wachs) |
| 6,65 % | PVP (Filmbildner) |

| Als Konservierungsmittel: | |
|---|---|
| 0,50 % | Phenoxyethanol |
| 0,22 % | Methylparaben |
| 0,08 % | Buthylparaben |
| 0,05 % | Ethylparaben |
| 0,05 % | Propylparaben |

| Als Pigment: | |
|---|---|
| 6,30 % | D&C Red No. 7 Calcium Lake |

Der zu den beiden Ausführungsbeispielen beschriebene Filmbildner PVP ist ein wasserdispergierbares Festigerharz und als solches ein hydrophobes Material mit einzelnen hydrophilen Gruppen.

## Patentansprüche

1. Kosmetische Masse für Lippenstift, Lidstift, Puder oder dergleichen umfassend
- Wasser mit einem Anteil von 30 bis 60%
- emulgierbares Hartwachs mit einem Anteil von 5 bis 50% und
- Polyalkohol als Lösungsvermittler mit einem Anteil von 15 bis 40% **gekennzeichnet durch** wasserlöslichen, bis wenigstens 100°C temperaturstabilen Filmbildner mit einem Anteil von 2 bis 20%.

2. Kosmetische Masse nach Anspruch 1, **dadurch gekennzeichnet, daß** die Hartwachse gebildet sind durch Stearamide Mea und/oder PVP/Eicosene Copolymer.

3. Kosmetische Masse nach Anspruch 1, **dadurch gekennzeichnet, daß** der Filmbildner gebildet ist durch Acrylates Copolymer, PVP, Hydroxyethylcellulose, vorzugsweise Diglycol/CHDM/Isophthalates/SIP Copolymer.

4. Kosmetische Masse nach Anspruch 1, **dadurch gekennzeichnet, daß** der Polyalkohol Butylene Glycol ist.

## Claims

1. A cosmetic composition for lipstick, eyeliner, powder or the like, comprising
- water at a percentage of 30 to 60 %;
- emulsifiable hard wax at a percentage of 5 to 50 %; and
- polyvalent alcohol as a solubilizer at a percentage of 15 to 40 %, **characterized by** a water-soluble film former which is thermally stable up to at least 100°C at a percentage of 2 to 20 %.

2. A cosmetic composition according to claim 1, wherein the hard waxes are formed by Stearamide MEA or PVP/Eicosone Copolymer.

3. A cosmetic composition according to claim 1, wherein the film former is formed by Acrylate Copolymer, PVP, Hydroxyethylcellulose, preferably Diglycol/CHDM/Isophthalates/SIP Copolymer.

4. A cosmetic composition according to claim 1, wherein the polyvalent alcohol is Butylene Glycol.

## Revendications

1. Masse cosmétique pour rouge à lèvres, crayon à paupières, poudre ou autres, comprenant
- de l'eau en une proportion de 30 à 60 %
- de la cire solide pouvant être émulsionnée en une proportion de 5 à 50 %
un polyalcool comme solvant en une proportion de 15 à 40 % en de préférence, **caractérisée par** un agent filmogène hydrosoluble stable au moins jusqu'à une température de 100° C en une proportion de 2 à 20 %.

2. Masse cosmétique selon la revendication 1, **caractérisé en ce que** les cires solides sont formées par le stéaramide Mea et/ou le copolymère PVP/Eicosène.

3. Masse cosmétique selon la revendication 1, **caractérisé en ce que** l'agent filmogène est formé par des copolymères d'acrylate, le PVP, l'hydroxyéthylcellulose, de préférence le copolymère de diglycol/CHDM/isophtalates/SIP.

4. Masse cosmétique selon la revendication 1, **caractérisé en ce que** le polyalcool est le butylène glycol.
